# DEMANDE DE BREVET EUROPEEN

(11) **EP 1 442 723 A1**
(43) Date de publication de la demande: **04.08.2004**
(21) Numéro de dépôt: 04354003.8
(22) Date de dépôt: 29.01.2004
(51) Int. Cl.: A61B 19/02, A61M 5/32, B65F 1/16

(54) **Conteneur pour déchets hospitaliers et médicaux et procédé de fermeture d'un tel conteneur**

(30) Priorité: 31.01.2003 FR 0301122
(71) Demandeur: Parolai Stil'Eco, 38330 Montbonnot Saint Martin (FR)
(72) Inventeur: Reynaud, Robert, 38190 Bernin (FR)
(74) Mandataire: Jouvray, Marie-Andrée

(57) **Abrégé**

Un conteneur destiné à collecter des déchets hospitaliers et médicaux comporte un récipient solidaire d'un couvercle (3) muni d'un capot (10) de fermeture pivotant. Le capot (10) comporte une languette (12) déformable permettant de rouvrir le capot (10) lorsque celui-ci est dans une position de fermeture provisoire. Des moyens de fermeture définitive rendent la languette (12) inaccessible, une fois le récipient rempli. Les moyens de fermeture définitive comportent au moins un crochet (14) destiné à s'encliqueter sous l'extrémité libre d'une paroi incurvée (7) du couvercle (3).

## Description

### Domaine technique de l'invention

L'invention concerne un conteneur destiné à collecter des déchets hospitaliers et médicaux comportant un récipient solidaire d'un couvercle muni d'un capot de fermeture pivotant, ledit capot comportant une languette déformable permettant de rouvrir le capot lorsque celui-ci est dans une position de fermeture provisoire, et des moyens de fermeture définitive rendant la languette inaccessible.

L'invention concerne également un procédé de fermeture d'un tel conteneur.

### État de la technique

Dans le domaine de la collecte des déchets dangereux, tels que les déchets hospitaliers, il est nécessaire de verrouiller définitivement le conteneur les collectant, une fois celui-ci rempli, de sorte que les déchets dangereux ne soient plus accessibles.

Des systèmes connus de fermeture pour des couvercles basculants ou pivotants autour d'un gond comportent généralement des moyens de verrouillage coopérant avec les parois du conteneur. Ainsi, le document EP-A1-0697344 décrit un conteneur constitué par une base solidarisée, au moyen d'une collerette, avec une partie supérieure comprenant un couvercle monté à pivotement autour d'un gond intégralement moulé. Le couvercle comprend une languette coopérant, dans un premier temps, avec une saillie de la partie supérieure du conteneur de manière à laisser la languette accessible, ce qui permet un blocage provisoire du couvercle. Dans un second temps, la languette coopère avec une butée rendant la languette inaccessible, pour un verrouillage définitif du couvercle. La mise en oeuvre d'un tel système de fermeture est compliqué.

### Objet de l'invention

L'objet de l'invention consiste à réaliser un conteneur à fermeture définitive fiable, sécurisée et simple à réaliser, ayant subit, au préalable, plusieurs cycles d'ouverture et de fermeture provisoire également faciles à réaliser.

Selon l'invention, ce but est atteint par le fait que :
- le couvercle comporte une paroi latérale prolongée perpendiculairement, à sa partie supérieure, par une bordure interne périphérique, dans laquelle est formée une cavité destinée à recevoir, pour la fermeture définitive du couvercle, la languette repliée selon un angle de l'ordre de +90° par rapport au plan du capot,
- la bordure interne se prolonge à l'intérieur du couvercle par une paroi incurvée,
- les moyens de fermeture définitive comportent au moins un crochet solidaire d'une paroi de verrouillage du capot et destiné à venir en butée sous l'extrémité libre de la paroi incurvée du couvercle, ladite paroi de verrouillage faisant saillie vers l'intérieur du couvercle sensiblement perpendiculairement au plan du capot.

Selon un développement de l'invention, la languette comporte des moyens de déformation destinés à replier la languette selon un angle compris entre environ - 90° et +90° par rapport au plan du capot.

Selon un mode de réalisation préférentiel, les moyens de déformation comportent une encoche délimitant, dans la languette, un premier élément fixe et solidaire du capot et un second élément destiné à pivoter par rapport au premier élément disposé dans le plan du capot.

L'invention a également pour but un procédé de fermeture d'un tel conteneur.

Selon l'invention, ce but est atteint par le fait que la fermeture définitive du conteneur consiste à :
- replier la languette selon un angle de l'ordre de +90° par rapport au plan du capot,
- introduire la languette repliée dans une cavité correspondante du couvercle,
- exercer une pression sur le capot pour amener au moins un crochet d'une paroi de verrouillage du capot sous l'extrémité libre d'une paroi incurvée du couvercle.

### Description sommaire des dessins

D'autres avantages et caractéristiques ressortiront plus clairement de la description qui va suivre de modes particuliers de réalisation de l'invention donnés à titre d'exemples non limitatifs et représentés aux dessins annexés, dans lesquels :
La figure 1 est une représentation en perspective d'un conteneur selon l'invention.
La figure 2 est une vue en coupe selon A-A du conteneur selon la figure 1.
La figure 3 représente une vue agrandie, en coupe selon A-A, des moyens de clipsage d'un récipient sur un couvercle du conteneur selon la figure 2.
Les figures 4 à 6 représentent, en coupe selon A-A, trois étapes successives de la fermeture provisoire d'un couvercle du conteneur selon la figure 1.
La figure 7 représente, en coupe selon A-A, un couvercle du conteneur selon la figure 1, en position ouverte.
Les figures 8 à 9 représentent, en coupe selon A-A, deux étapes successives de la fermeture définitive d'un capot du couvercle selon la figure 4.

### Description de modes particuliers de réalisation.

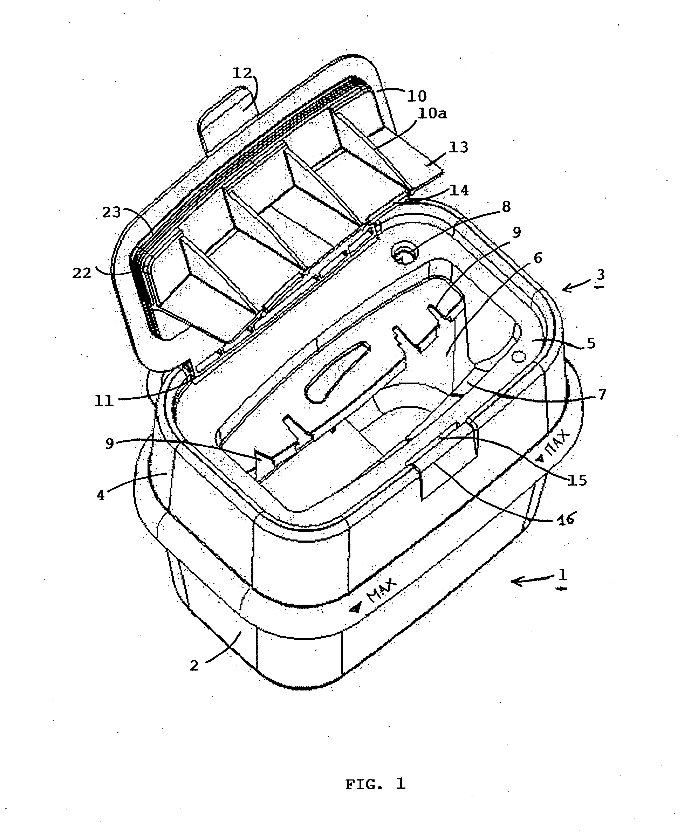

Sur les figures 1 et 2, un conteneur 1 destiné à collecter des déchets hospitaliers et médicaux, comporte un récipient 2 rendu solidaire d'un couvercle 3 par tout type de moyens appropriés. Par déchets hospitaliers et médicaux, on entend des éléments susceptibles d'être infectieux, tels que des éléments coupants, tranchants ou piquants, par exemple des seringues. Le récipient 2 a une forme sensiblement trapézoïdale et le couvercle 3 comporte une paroi latérale 4 prolongée perpendiculairement, à sa partie supérieure, par une bordure interne 5 périphérique.

La bordure interne 5 comporte des côtés opposés deux à deux, de manière à former une ouverture 6 par laquelle sont introduits, dans le récipient 2, les déchets. Un des côtés de la bordure interne 5 est prolongé, à l'intérieur du couvercle 3, par une paroi incurvée 7 empêchant le reflux des déchets vers l'extérieur du récipient 2. La bordure interne 5 peut également comporter un orifice borgne 8 destiné à être étanche et à poser les étuis des seringues ainsi qu'une pluralité de fentes 9, de différentes formes entourées de rebords anti-reflux et destinées à dissocier les éléments des déchets (figure 1), par exemple à séparer une aiguille du reste d'une seringue.

Le couvercle 3 est muni d'un capot de fermeture 10 pivotant autour d'un gond 11 disposé longitudinalement sur un des côtés de la bordure interne 5, de préférence, le côté opposé au côté prolongé par la paroi incurvée 7. Le capot 10 est destiné à être fermé et rouvert provisoirement pendant la durée de remplissage du récipient 2, puis il est fermé définitivement, une fois le récipient 2 rempli. Le récipient 2 comporte un indicateur de niveau de remplissage pour déterminer la fermeture définitive du couvercle 3. Sur la figure 1, l'indicateur de niveau de remplissage correspond à la mention Max disposée sur une bordure périphérique externe du couvercle 3.

Le capot 10 comporte une base plane, une languette déformable 12 et une paroi de verrouillage 13. La paroi de verrouillage 13 fait saillie vers l'intérieur du couvercle 3 sensiblement perpendiculairement au plan du capot 10 (figure 1) et est munie à son extrémité libre d'au moins un crochet. Sur la figure 1, la paroi de verrouillage comporte à son extrémité libre deux crochets 14 et une pluralité de nervures de rigidification 10a sont disposées entre la base plane du capot 10 et la paroi de verrouillage 13.

La languette 12 est solidaire d'un bord du capot 10 et est agencée, dans un premier temps, pour rouvrir le capot 10 lorsqu'il est dans une position de fermeture provisoire. Sur la figure 1, la languette 12 est contenue dans le plan du capot 10, mais peut être repliée selon un angle de +90° par rapport au plan du capot 10, de manière à être logée, lors de la fermeture définitive du couvercle 3, dans une cavité 15 formée dans la bordure interne 5 (figure 2).

La cavité 15 possède, de préférence, une section sensiblement en forme de U disposée du côté de la bordure interne 5 prolongée par la paroi incurvée 7. Elle comporte des première et seconde parois longitudinales 15a et 15b adjacentes, respectivement, à la paroi incurvée 7 et à la paroi latérale 4. Sur la figure 2, la seconde paroi longitudinale 15b présente une hauteur supérieure à celle de la première paroi longitudinale 15a, de sorte que son extrémité supérieure est prolongée par un rebord incliné 16 rejoignant la paroi latérale 4 du couvercle 3. La languette 12 comporte, de préférence, des moyens de déformation destinés à la replier selon un angle compris entre environ -90° et +90° par rapport au plan du capot 10, l'orientation de l'extrémité libre de la languette 12 dépendant de la fermeture provisoire ou définitive du couvercle. Sur la figure 4, une encoche 17, en forme de V renversé, permet de replier la languette 12 de ±90° par rapport au plan du capot 10. L'encoche 17 délimite, dans la languette 12, un premier élément 12a fixe et solidaire du capot 10 et un second élément 12b destiné à pivoter selon un angle compris entre environ -90° et +90° par rapport au premier élément 12a restant dans le plan du capot 10. Le second élément 12a de la languette 12 comporte, sur sa partie inférieure, un épaulement 18 adjacent à l'encoche 17 et destiné à arrêter le pivotement du capot lors d'une fermeture provisoire.

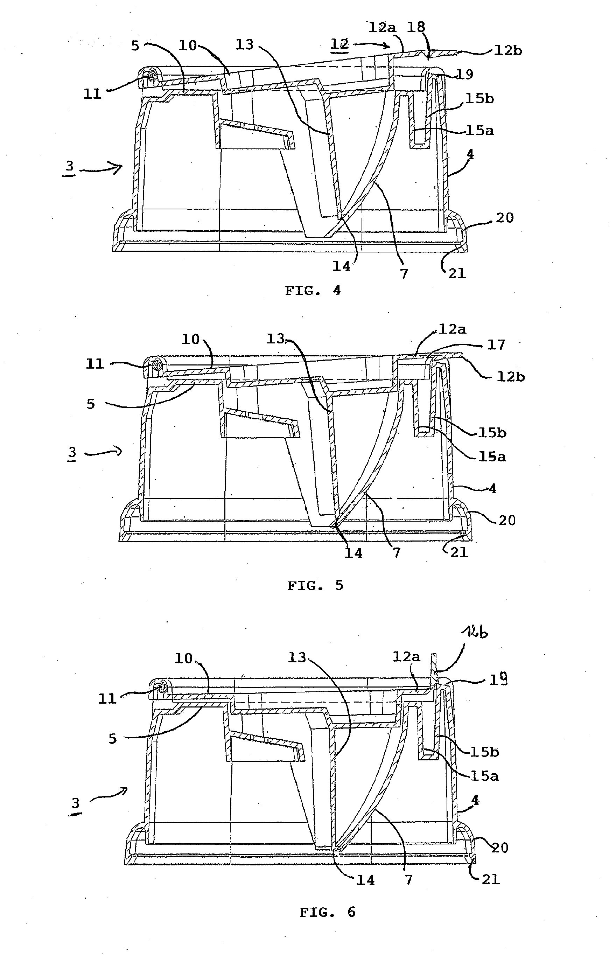

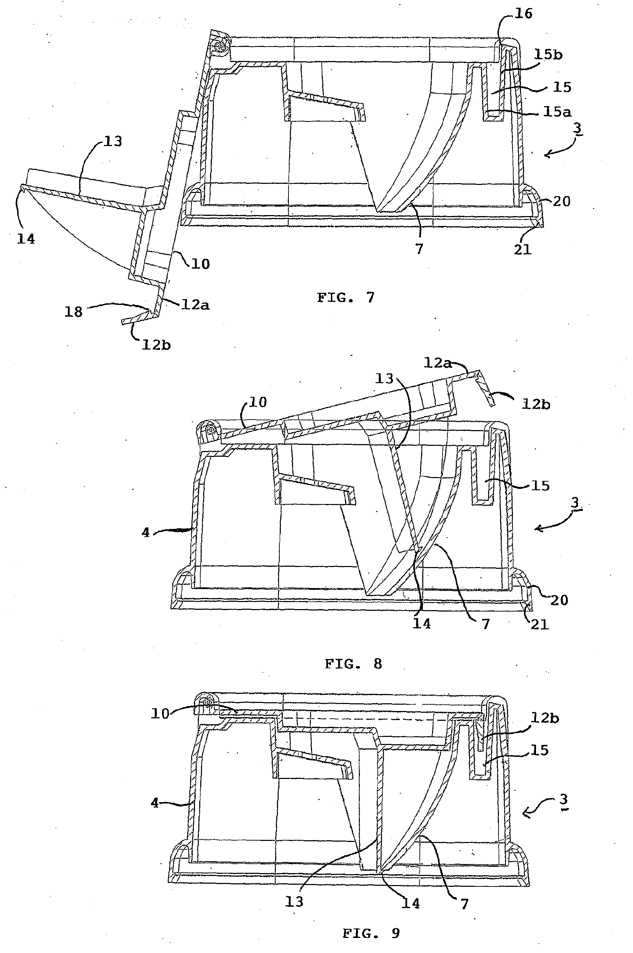

Comme représentée sur les figures 4 à 6, la fermeture provisoire du couvercle 3 consiste à faire basculer le capot 10 vers la bordure interne 5 du couvercle 3, de manière à introduire la paroi de verrouillage 13 dans l'ouverture 6 (figure 4) jusqu'à ce que l'épaulement 18 de la languette 12 vienne en butée sur le rebord incliné 16 du couvercle 3 (figure 5). En fermant le capot 10, les crochets 14 disposés à l'extrémité libre de la paroi de verrouillage 13 touchent la paroi incurvée 7 qui se déforme légèrement. Le second élément 12b de la languette 12 se replie selon un angle de l'ordre de -90° par rapport au premier élément 12a, de sorte que l'encoche 17 coopère avec le rebord incliné 16 du couvercle 3 pour permettre à l'épaulement 18 de venir en butée sur le rebord interne 16 (figure 6). L'extrémité libre de la languette 12 est, alors, dirigée vers le haut et peut être facilement saisie pour rouvrir le capot 10 et introduire des déchets. La fermeture provisoire du couvercle est simple à réaliser puisque l'arrêt du pivotement du capot est réalisé spontanément par l'épaulement 18.

Une fois le récipient 2 rempli, le couvercle 3 doit être fermé définitivement. La fermeture définitive consiste, alors, à replier la languette 12 selon un angle de l'ordre de +90° par rapport au plan du capot 10 et plus particulièrement à replier le second élément 12b de la languette 12 par rapport au premier élément 12a (figure 7). Il faut, ensuite, faire basculer le capot 10 vers la bordure interne 5 du couvercle 3, de sorte que la paroi de verrouillage 13 s'introduise dans l'ouverture 6 (figure 8) et que la languette 12 se loge dans la cavité 15 (figure 9). Le chemin parcouru par l'extrémité libre des crochets 14 de la paroi de verrouillage 13, pendant le basculement du capot 10, suit, de préférence, la courbure de la paroi incurvée de la bordure interne 5. Une pression doit, alors, être exercée sur la surface extérieure du capot de fermeture 10 jusqu'à ce que les crochets 14 solidaires de la paroi de verrouillage 13 se placent sous l'extrémité libre de la paroi incurvée 7 de la bordure interne 5 (figure 9). Les crochets 14 assurent un verrouillage positif du capot 10 et la languette 12 est rendue inaccessible, de manière à interdire l'ouverture du capot.

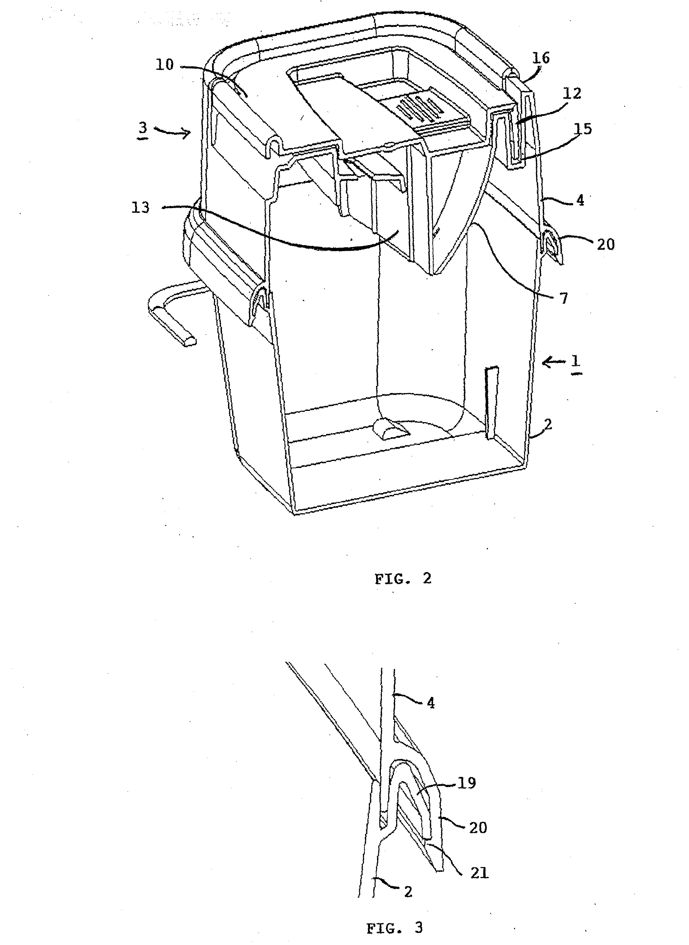

Selon un mode particulier de réalisation, le couvercle 3 est rendu solidaire du récipient 2 par un système d'encliquetage. Ainsi, la périphérie supérieure du récipient 2 comporte un rebord 19, en forme de V renversé et faisant saillie vers l'extérieur, de manière à former, avec la paroi du récipient 2, un espace dans lequel s'engage l'extrémité libre de la paroi latérale 4 du couvercle 3 (figure 3). La paroi latérale 4 est, ainsi, prolongée à sa partie inférieure, par une bordure externe périphérique 20 dont l'extrémité libre est munie d'une saillie interne 21 destinée à bloquer définitivement l'extrémité libre du rebord 19 lorsque le couvercle 3 est disposé sur le récipient 2. Le conteneur 1 est ainsi scellé définitivement, lorsque le couvercle 3 est en position de fermeture définitive.

La base plane du capot 10 peut également être prolongée, sur sa partie inférieure, par une bordure 22 d'étanchéité comportant des nervures de maintien 23 longitudinales destinées à améliorer l'étanchéité du couvercle, une fois celui-ci fermé définitivement (figure 1).

L'avantage d'un conteneur selon l'invention est que le scellement définitif du conteneur est fiable et sécurisé puisque les crochets 14 rendent la réouverture du capot impossible. Le scellement est également réalisé en un nombre d'étapes simples et limitées. De plus, ce type de scellement définitif est adaptable sur des récipients de volumes différents. Ainsi, un même couvercle peut être adapté sur des récipients de 1,5 L, de 3 L ou de 8 L et un autre couvercle de section plus large peut être monté sur des récipients de 5L ou de 11L.

## Revendications

1. Conteneur destiné à collecter des déchets hospitaliers et médicaux comportant un récipient (2) solidaire d'un couvercle (3) muni d'un capot (10) de fermeture pivotant, ledit capot (10) comportant une languette (12) déformable permettant de rouvrir le capot (10) lorsque celui-ci est dans une position de fermeture provisoire, et des moyens de fermeture définitive rendant la languette (12) inaccessible, conteneur **caractérisé en ce que** :
- le couvercle (3) comporte une paroi latérale (4) prolongée perpendiculairement, à sa partie supérieure, par une bordure interne (5) périphérique, dans laquelle est formée une cavité (15) destinée à recevoir, pour la fermeture définitive du couvercle (3), la languette (12) repliée selon un angle de l'ordre de +90° par rapport au plan du capot (10),
- la bordure interne (5) se prolonge à l'intérieur du couvercle (3) par une paroi incurvée (7),
- les moyens de fermeture définitive comportent au moins un crochet (14) solidaire d'une paroi de verrouillage du capot (10) et destiné à venir en butée sous l'extrémité libre de la paroi incurvée (7) du couvercle (3), ladite paroi de verrouillage (13) faisant saillie vers l'intérieur du couvercle (3) sensiblement perpendiculairement au plan du capot (10).

2. Conteneur selon la revendication 1, **caractérisé** en que la cavité (15) est formée dans la bordure interne (5), entre la paroi latérale (4) et la paroi incurvée (7) du couvercle (3).

3. Conteneur selon l'une des revendications 1 et 2, **caractérisé en ce que** la languette (12) comporte des moyens de déformation destinés à replier la languette (12) selon un angle compris entre environ -90° et +90° par rapport au plan du capot (10).

4. Conteneur selon la revendication 3, **caractérisé** en que les moyens de déformation comportent une encoche (17) délimitant, dans la languette (12), un premier élément (12a) fixe et solidaire du capot (10) et un second élément (12b) destiné à pivoter par rapport au premier élément (12a) disposé dans le plan du capot.

5. Conteneur selon la revendication 4, **caractérisé** en que le second élément de la languette (12) comporte un épaulement (18) adjacent à l'encoche (17) et destiné à arrêter, en position de fermeture provisoire, le pivotement du capot (10).

6. Conteneur selon l'une quelconque des revendications 1 à 5, **caractérisé** en que le chemin parcouru par l'extrémité libre du crochet (14) de la paroi de verrouillage (13) suit la courbure de la paroi incurvée (7) de la bordure interne (5) périphérique, lors d'une fermeture définitive du capot (10).

7. Conteneur selon l'une quelconque des revendications 1 à 6, **caractérisé en ce que** la paroi latérale (4) du couvercle est prolongée, à sa partie inférieure, par une bordure externe (20) périphérique dont l'extrémité libre est munie d'une saillie interne (21) destinée à bloquer définitivement un rebord (19) disposé à la périphérie supérieure du récipient (2) et faisant saillie vers l'extérieur.

8. Procédé de fermeture d'un conteneur selon l'une quelconque des revendications 1 à 7, destiné à collecter des déchets hospitaliers comportant un récipient (2) solidaire d'un couvercle (3) muni d'un capot (10) de fermeture pivotant, ledit capot (10) comportant une languette (12) déformable permettant de rouvrir le capot (10) lorsque celui-ci est dans une position de fermeture provisoire, et des moyens de fermeture définitive rendant la languette (12) inaccessible,
**caractérisé** en que la fermeture définitive du conteneur (1) consiste à :
- replier la languette (12) selon un angle de l'ordre de +90° par rapport au plan du capot (10),
- introduire la languette (12) repliée dans une cavité (15) correspondante du couvercle (3),
- exercer une pression sur le capot (10) pour amener au moins un crochet (14) d'une paroi de verrouillage (13) du capot (10) sous l'extrémité libre d'une paroi incurvée (7) du couvercle.

9. Procédé de fermeture selon la revendication 8 **caractérisé en ce que**, dans la position de fermeture provisoire du capot (10), la languette (12) déformable est repliée selon un angle de l'ordre de -90° par rapport au plan du capot (10), de manière à positionner un épaulement (18) de la languette (12) en butée sur la bordure interne (5) du couvercle.
